# EUROPÄISCHE PATENTSCHRIFT

(11) **EP 0 648 779 B1**
(45) Veröffentlichungstag und Bekanntmachung des Hinweises auf die Patenterteilung: **05.03.1997**
(21) Anmeldenummer: 94250179.2
(22) Anmeldetag: 07.07.1994
(51) Int. Cl.: C07J 41/00, A61K 31/565

(54) **Neue 11-Benzaldoxim-17beta methoxy-17alpha-methoxymethyl-estradien-Derivate, Verfahren zu ihrer Herstellung und diese Verbindungen enthaltende Arzneimittel**
New 11-benzaldehyde oxime, 17-beta methoxy, 17 alpha methoxymethyl derivates of estradiene, a process for their preparation and pharmaceutical compositions containing them
Nouveaux dérivés 11-benzaldoxime, 17-bêta-méthoxy, 17 alpha-méthoxy méthyle de estradiène, un procédé pour leur préparation et les compositions pharmaceutiques les contenant

(30) Priorität: 20.09.1993 DE 4332284
(43) Veröffentlichungstag der Anmeldung: 19.04.1995
(73) Patentinhaber: JENAPHARM GmbH, D-07745 Jena (DE)
(72) Erfinder: Schubert, Gerd, Dr., D-07743 Jena (DE); Kaufmann, Günter, Dr., D-07743 Jena (DE); Sobeck, Lothar, Dr., D-07743 Jean (DE); Oettel, Michael, Prof.Dr., D-07743 Jena (DE); Elger, Walter, Dr., D-14195 Berlin (DE); Kurischko, Anatoli, Dr., D-07745 Jena (DE)
(74) Vertreter: Wablat, Wolfgang, Dr.Dr.

(56) Entgegenhaltungen:
- EP-A- 0 190 759
- EP-A- 0 411 733
- EP-A- 0 648 778

## Beschreibung

Die Erfindung betrifft neue 11-Benzaldoxim-estradien-Derivate, Verfahren zu ihrer Herstellung und diese Verbindungen enthaltende Arzneimittel.

11β-substituierte Phenyl-estratriene sind bereits bekannt. Die Herstellung von 11β-Aryl-17α-propinyl-estra-4,9-dienen ist z. B. in der Patentschrift EP 057 115, die Umsetzung von 11β-(4-Formylphenyl)-estra-4,9-dien-3-onen mit Hydroxylaminen in der Patentschrift DE 3 504 421 bereits beschrieben. In dem angegebenen Verfahren wird sowohl die 11β-Formylphenylgruppe als auch die 3-Ketogruppe oximiert. Außerdem entstehen am C-3 syn-und anti-Isomere. Über die Wirkung der beschriebenen Verbindungen ist bisher nichts bekannt. Bekannt sind aus der EP 0 411 733 ferner 11β-Aryl-gona-4,9-dien-3-one, welche in der 4-Stellung des 11β-phenylrestes verschiedene substituenten tragen. Oxime werden jedoch nicht offenbart.

Progesteron wird während des Zyklus und in der Gravidität in großen Mengen vom Ovar und der Plazenta sezerniert. Seine regulatorische Bedeutung ist vermutlich nicht in allen Aspekten geklärt.

Gesichert ist, daß Progesteron im Zusammenwirken mit Oestrogenen die zyklischen Veränderungen der Uterusschleimhaut im Menstruationszyklus und der Gravidität bewirkt. Unter dem Einfluß erhöhter Progesteronspiegel nach der Ovulation wird die Uterusschleimhaut in einen Zustand überführt, der die Einnistung eines Embryos (Blastozyste) zuläßt. Die Erhaltung der Gewebe, in denen sich der wachsende Embryo ausbreitet, ist ebenfalls progesteronabhängig.

In der Gravidität findet eine dramatische Veränderung der muskulären Funktion des Uterus statt. Der gravide Uterusmuskel reagiert stark abgeschwächt oder gar nicht auf hormonale und mechanische Reize, die außerhalb der Gravidität Wehen auslösen. Es kann kein Zweifel daran bestehen, daß hierbei Progesteron eine Schlüsselrolle spielt, obwohl in manchen Phasen der Gravidität, z. B. unmittelbar vor der Geburt, eine hohe Reaktionsbereitschaft bei extrem hohen Blutspiegeln von Progesteron besteht.

Andere typische Vorgänge der Schwangerschaft sind ebenfalls Ausdruck der sehr hohen Progesteronspiegel. Der Aufbau der Brustdrüsen und der feste Verschluß des Muttermundes bis in die Nähe des Geburtstermins sind hierfür Beispiele.

Progesteron ist in subtiler Weise an der Steuerung von Ovulationsvorgängen beteiligt. Es ist bekannt, daß Progesteron in hohen Dosierungen antiovulatorische Eigenschaften besitzt. Diese resultieren aus einer Hemmung der hypophysären Gonadotropinsekretion, die Voraussetzung für die Reifung eines Follikels und dessen Ovulation ist. Andererseits ist erkennbar, daß die vergleichsweise geringe Progesteronsekretion des reifenden Follikels eine aktive Rolle für die Vorbereitung und Auslösung der Ovulation spielt. Hierbei spielen hypophysäre Mechanismen (zeitlich begrenzter sog. positiver feed back des Progesterons auf Gonadotropinsekretion) erkennbar eine wichtige Rolle (Loutradie, D.; Human Reproduction 6, 1991, 1238-1240).

Weniger gut analysiert sind Funktionen des Progesterons im reifenden Follikel und Gelbkörper selbst, an deren Existenz kein Zweifel besteht. Letztlich sind auch hier stimulierende und hemmende Effekte auf endokrine Follikel- und Gelbkörperfunktion anzunehmen.

Eine bedeutende Rolle des Progesterons und der Progesteronrezeptoren ist auch in pathophysiologischen Prozessen anzunehmen. Progesteronrezeptoren sind in Endometrioseherden, aber auch in Tumoren des Uterus, der Mamma und des ZNS (Meningeome) nachgewiesen. Die Rolle dieser Rezeptoren für das Wachstumsverhalten dieser pathologisch relevanten Gewebe ist nicht notwendigerweise an das Vorliegen von Progesteronspiegeln im Blut gebunden. Es ist erweisen, daß Substanzen, die als Progesteronantagonisten charakterisiert sind, RU 486 = Mifepriston (EP-0 057 115) und ZK 98299 = Onapriston (DE-OS-35 04 421) in diesen Geweben auch dann tiefgreifende Funktionsänderungen auslösen, wenn vernachlässigbar geringe Progesteronspiegel im Blut vorliegen. Es erscheint möglich, daß hierbei Änderungen von Transskriptionswirkungen des nicht von Progesteron besetzten Progesteronrezeptors durch die Antagonisten eine wichtige Rolle spielen (Chwalisz, K. et al., Endocrinology, 129, 317-322, 1991).

Die Wirkungen von Progesteron in Geweben der Genitalorgane und anderen Geweben erfolgen durch Interaktion mit dem Progesteronrezeptor. In der Zelle bindet sich Progesteron mit hoher Affinität an seinen Rezeptor. Hierdurch werden Veränderungen des Rezeptorproteins bewirkt: Konformationsveränderungen, Dimerisierung von 2 Rezeptoreinheiten zu einem Komplex, Entblößung der DNA-Bindungsstelle des Rezeptors durch Abdissoziierung eines Proteins (HSP 90), Bindung an Hormon-responsible Elemente der DNA. Letztlich wird die Transskription bestimmter Gene reguliert. (Gronemeyer, H. et al., J. Steroid Biochem. Molec. Biol. 41, 3-8, 1992).

Die Wirkung von Progesteron oder von Progesteronantagonisten ist nicht nur von ihrer Konzentration im Blut abhängig. Die Konzentration von Rezeptoren in der Zelle ist ebenfalls stark reguliert. Oestrogene stimulieren die Synthese von Progesteronrezeptoren in den meisten Geweben. Progesteron hemmt die Synthese von Oestrogenrezeptoren und diejenige seines eigenen Rezeptors. Vermutlich sind es diese und andere interaktive Beziehungen zwischen Oestrogenen und Gestagenen, die erklären können, warum Gestagene und Antigestagene oestrogenabhängige Vorgänge beeinflussen können ohne daß sie vom Oestrogenrezeptor gebunden werden. Diese Beziehungen sind naturgemäß von großer Bedeutung für die therapeutische Anwendung von Antigestagenen. Diese Substanzen erscheinen geeignet, gezielt in Reproduktionsvorgänge der Frau einzugreifen, z. B. postovulatorisch, um die Nidation zu verhindern, in der späteren Gravidität, um Reaktionsbereitschaft des Uterus für Prostaglandine und Oxytocin zu erhöhen oder um eine Eröffnung und Erweichung ("Reifung") der Cervix zu erreichen.

Antigestagene hemmen bei verschiedenen subhumanen Primatenspezies die Ovulation. Der Mechanismus dieser Wirkung ist nicht eindeutig geklärt. Diskutiert werden neben einer Hemmung der Gonadotropinsekretion auch ovarielle Mechanismen durch Störung para- und autokriner Funktionen des Progesteron im Ovar.

Antigestagene haben die Fähigkeit, die Effekte von Oestrogenen zu modulieren oder abzuschwächen, obwohl sie selbst überwiegend keine Affinität zum Oestrogenrezeptor auf cytoplasmatischer Ebene besitzen und obwohl sie einen Anstieg der Oestrogenrezeptorkonzentration bewirken können. Von entsprechenden Effekten kann in Endometrioseherden bzw. in Tumorgeweben, das mit Oestrogen- und Progesteronrezeptoren ausgestattet ist, eine günstige Beeinflussung von Krankheitszuständen erwartet werden. Besondere Vorteile für die günstige Beeinflussung von Krankheitszuständen wie Endometriose könnten dann gegeben sein, wenn zu den Hemmeffekten eines Antigestagens durch die Wirkung im Gewebe eine Hemmung der Ovulation treten würde. Mit der Hemmung der Ovulation würde auch ein Teil der ovariellen Hormonproduktion und damit der auf diesen Anteil entfallende Stimulationseffekt auf das pathologisch veränderte Gewebe entfallen. Es wäre wünschenswert, bei Vorliegen einer schweren Endometriose die Ovulation zu hemmen und die sonst ständig im Umbau befindlichen Gewebe des Genitaltraktes reversibel in einen ruhenden Zustand zu versetzen.

Auch für die Kontrazeption wird ein Verfahren diskutiert, bei dem eine Antigestagenbehandlung die Ovulation unterdrückt, und durch eine anschließende Gestagenbehandlung die sekretorische Transformation des Endometriums induziert wird, so daß aus Behandlungstagen mit Antigestagenen und Gestagenen und behandlungsfreien Tagen ein ca. 28-tägiger Zyklus mit regelmäßigen Entzugsblutungen resultiert (Baulieu, E.E., Advances in Contraception 7, 345-51, 1991).

Antigestagene können unterschiedliche hormonale und antihormonale Eigenschaften besitzen. Von besonderer therapeutischer Relevanz sind hierbei antiglucocorticoide Eigenschaften. Diese sind für therapeutische Anwendungen, bei denen die Hemmung der Progesteronrezeptoren im Vordergrund der Therapie steht, nachteilig, da sie bei den therapeutisch erforderlichen Dosierungen unerwünschte Nebenwirkungen verursachen, die Applikation einer therapeutisch sinnvollen Dosis verhindern, oder zum Abbruch der Behandlung führen können. Die teilweise oder vollständige Reduktion antiglucocorticoider Eigenschaften ist eine wichtige Voraussetzung für die Therapie mit Antigestagenen, insbesondere für diejenigen Indikationen, die eine über Wochen oder Monate dauernde Behandlung erfordern.

Aufgabe der vorliegenden Erfindung ist es, neue 11β-Benzaldoximestra-4,9-dien-Derivate der allgemeinen Formel I 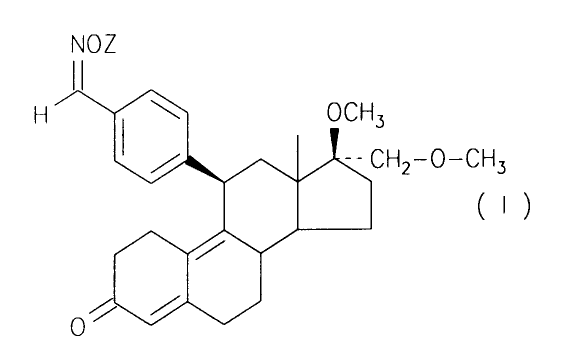 und ihre pharmazeutisch annehmbaren Salze sowie ein Verfahren zu ihrer Herstellung zur Verfügung zu stellen. Eine weitere Aufgabe ist es, Arzneimittel, die eine Verbindung der allgemeinen Formel I oder deren pharmazeutisch annehmbares Salz erhalten, zur Verfügung zu stellen.

In der allgemeinen Formel I, 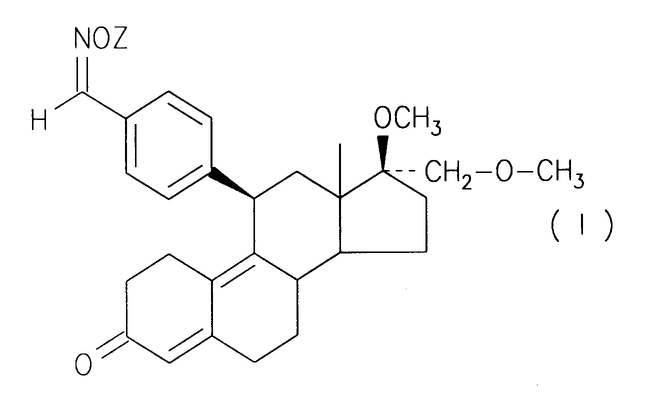 steht Z für -CO-CH₃, -CO-O-C₂H₅, -CO-NH-Phenyl, -CO-NH-C₂H₅, -CO-C₂H₅ oder -CO-Phenyl.

Bevorzugte erfindungsgemäße Verbindungen sind:
11β-[4-(Acetoximinomethyl)phenyl]-17β-methoxy-17α-methoxymethyl-estra-4,9-dien-3-on,
11β-{4-[(Ethoxycarbonyl)oximinomethyl]phenyl}-17β-methoxy-17α-methoxymethyl-estra-4,9-dien-3-on,
11β-{4-[(Ethylaminocarbonyl)oximinomethyl]phenyl}-17β-methoxy-17α-methoxymethyl-estra-4,9-dien-3-on,
17β-Methoxy-17α-methoxymethyl-11β-{4-[(phenylaminocarbonyl)oximinomethyl]phenyl}-estra-4,9-dien-3-on,
11β-[4-(Propionyloximinomethyl)phenyl]-17β-methoxy-17α-methoxymehyl-estra-4,9-dien-3-on, und
11β-[4-(Benzoyloximinomethyl)phenyl]-17β-methoxy-17α-methoxymethyl-estra-4,9-dien-3-on.

Die Erfindung betrifft ferner ein Verfahren zur Herstellung der Verbindungen nach der allgemeinen Formel I und deren pharmazeutisch annehmbaren Salze, das dadurch gekennzeichnet ist, daß man eine Verbindung der allgemeinen Formel II, 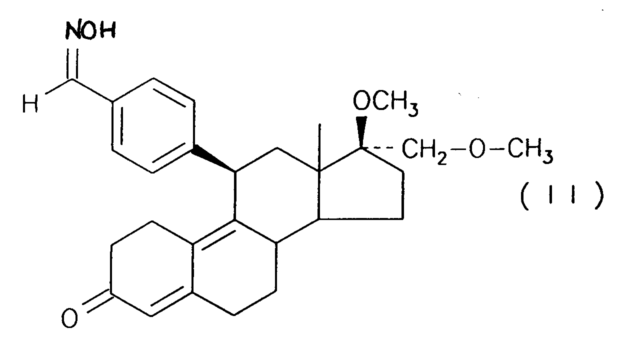 mit der Gruppe Z verestert und gegebenenfalls die erhaltene Verbindung in ein pharmazeutisch annehmbares Salz überführt.

Die Herstellung von Verbindungen der allgemeinen Formel I mittels Veresterung oder Urethan-Bildung kann in an sich bekannter Weise mit Acylierungsmitteln wie Säureanhydriden oder Säurechloriden in Gegenwart von Basen, vorzugsweise Pyridin und die Urethan-Bildung durch Reaktion mit Alkyl- oder Arylisocyanaten in inerten Lösungsmitteln, vorzugsweise Toluol oder durch Reaktion von Carbamoylchloriden in Gegenwart von Basen, vorzugsweise Triethylamin durchgeführt werden.

Die Herstellung der Ausgangsverbindung der allgemeinen Formel II erfolgt aus dem 5α,10α-Epoxid III 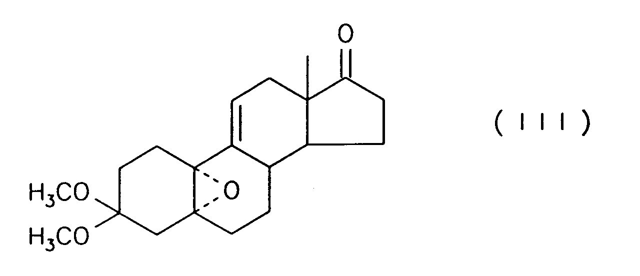 [vgl. z.B. Nédélec Bull. Soc. chim. France (1970), 2548].

Die Einführung des Phenylrestes in die 11β-Stellung unter Ausbildung einer Δ9(10),5α-Hydroxy-Struktur IV wird durch Cu(I)-Salz katalysierte Grignard-Reaktion (Tetrahedron Letters 1979, 2051) mit einem p-Brombenzaldehydketal, vorzugsweise dem p-Brombenzaldehyddimethylketal bei Temperaturen zwischen 0°C und 30°C erreicht.

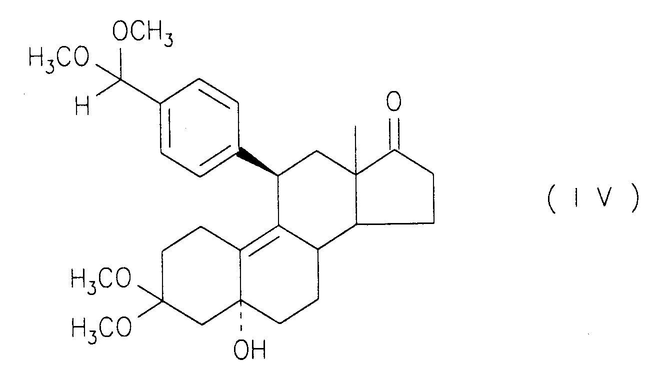

Die Einführung der Gruppierung -CH₂-O-CH₃ in der 17-Position erfolgt nach dem an sich bekannten Weg über das Spiroepoxid V durch Umsetzung mit Trimethylsulfoniumjodid und Kalium-tert.-butanolat in Dimethylsulfoxid [Hübner et.al.; J. prakt. Chem. 314, 667 (1972); Arzneim. Forsch. 30, 401 (1973)] und 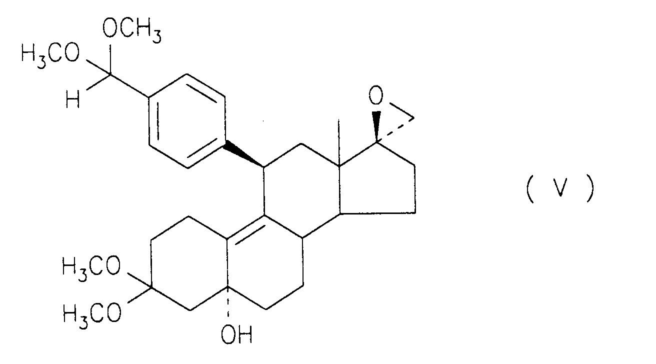 anschließende Ringöffnung mit Alkoholaten [Ponsold et.al.; Z. Chem. 11, 106 (1971)]. Die dabei entstehenden 17α-CH₂-O-CH₃-Verbindungen VI 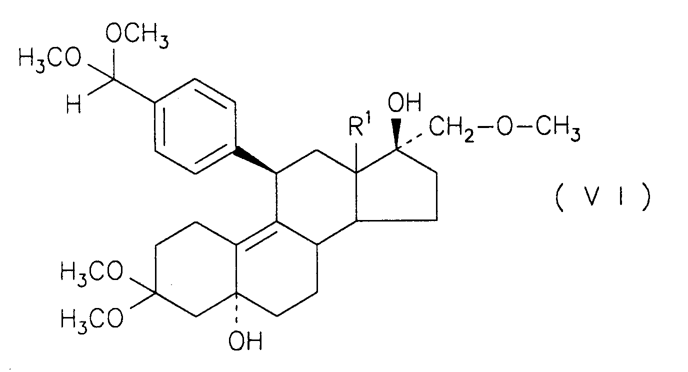 lassen sich entweder durch saure Hydrolyse, vorzugsweise mit p-Toluolsulfonsäure in Aceton (Teutsch et.al. DE 2801416) zu den entsprechenden Aldehyden spalten oder nach Verethern der freien Hydroxylgruppen mit Alkylhalogeniden in Gegenwart von Kalium-tert.-butanolat zunächst in die 5α,17β-Diether überführen (Kasch et.al. DD 290 893), die dann durch saure Hydrolyse, vorzugsweise mit p-Toluolsulfonsäure in Aceton, zu den entsprechenden Aldehyden umgewandelt werden, wonach dann die so erhaltenen Aldehyde durch Umsetzung mit Hydroxylamin in die Verbindungen der allgemeinen Formel II überführt werden.

Gegebenenfalls wird die erhaltene erfindungsgemäße Verbindung der allgemeinen Formel I in ein Säureadditionssalz, vorzugsweise in ein Salz einer physiologisch verträglichen Säure überführt. Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beisplielweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wäßrige Lösungen von Säureadditionssalzen der Verbindung der Formel I in einer wäßrigen Säurelösung hergestellt werden.

Die Säureadditionssalze der Verbindungen der allgemeinen Formel I können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, subcutanen, intravenösen oder intramuskulären Applikation, die neben üblichen Träger- und Verdünnungsmitteln eine Verbindung der allgemeinen Formel I oder deren Säureadditionssalz als Wirkstoff enthalten.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Die erfindungsgemäßen 11β-substituierten Benzaldoximestra-4,9-diene sind antigestagen wirkende Substanzen, die bei überlegener Wirkung in vivo (vgl. Tab. 2) eine im Vergleich zu RU 486 deutlich reduzierte antiglucocorticoide Aktivität besitzen, nachgewiesen durch die verminderte Glucocorticoid-Rezeptorbindung (vgl. Tab. 1)

**Tabelle 1**

| Rezeptorbindung von ausgewählten, unter Beispiel 1 und 2 aufgeführten Substanzen | |
|---|---|
| Verbindung nach Beispiel | relative molare Bindungsaffinität (RBA) [%] zumGlucocorticoid-Rezeptor (Dexamethason=100%) |
| 1 (J914) | 73 |
| 2 (J900) | 66 |

| z. Vergleich | |
|---|---|
| RU 486 (Mifepristone) | 685 |
| ZK 98299 (Onapristone) | 39 |

Diese Kombination von Eigenschaften läßt von den erfindungsgemäßen Antigestagenen eine überlegene Hemmung von Progesteron, bei gleichzeitig reduzierter antiglucocorticoider Aktivität erwarten. Dieser Vorteil ist besonders im Hinblick auf Indikationen relevant, die wegen der Dauer der Anwendung eine besonders gute Verträglichkeit erfordern. Im Zyklus wird das Uterusgewicht entscheidend von zirkulierenden Oestrogenen bestimmt. Reduzierte Uterusgewichte sind Ausdruck einer Hemmung dieser Funktion der Oestrogene. Die festgestellte, RU 486 überlegene Hemmung der Uterusgewichte im Zyklus des Meerschweinchens, ist ein Hinweis auf (indirekte) antioestrogene Eigenschaften der erfindungsgemäßen Verbindungen. Von entsprechenden Effekten ist eine besonders günstige Beeinflussung von pathologisch veränderten Geweben zu erwarten, in denen Oestrogene Wachstumsimpulse vermitteln (Endometrioseherde, Myome, Mamma- und Genitalcarcinome, benigne Hypertrophie der Prostata).

**Tabelle 2**

| Frühabortive Wirkung von RU 486 und J 914 (Beispiel 1) und J 900 (Beispiel 2) bei der Ratte nach subkutaner Applikation vom 5. - 7. Graviditätstag (Applikation 0,2 ml/Tier/Tag in Benzylbenzoat/Rizinusöl [1 + 4 v/v]) | | | | |
|---|---|---|---|---|
| Gruppe, Substanz | Dosis (mg/Tier/Tag) | komplette Graviditätshemmung⁺ | | ED 50 ⁺⁺ mg/Tier/Tag) |
| | | N* /N | % | |
| Vehikel | - | 0/25 | 0 | - |
| RU 486 | 3,0 | 5/5 | 100 | 1,1 |
| | 1,0 | 2/5 | 40 | |
| | 0,3 | 0/5 | 0 | |
| J 900 | 1,0 | 9/10 | 90 | 0,6 |
| | 0,3 | 0/5 | 0 | |
| J 914 | 3,0 | 5/5 | 100 | 0,6 |
| | 1,0 | 7/10 | 70 | |
| | 0,3 | 1/5 | 20 | |
| | 0,1 | 0/6 | 0 | |

| | | | | |
|---|---|---|---|---|
| + - leere Uteri; | | | | |
| N - Zahl der besamten Weibchen; | | | | |
| N* - Zahl der nichtgraviden Weibchen; | | | | |
| ++ - graphische Bestimmung | | | | |

Die folgenden Beispiele erläutern die Erfindung

### Beispiele

### Beispiel 1

180 mg 11β-[4-(Hydroximinomethyl)phenyl]-17β-methoxy-17α-methoxymethyl-estra-4,9-dien-3-on werden in 5 ml Acetanhydrid/Pyridin (1:1) acetyliert. Nach Zugabe von Wasser wird dreimal mit Essigester extrahiert, die organische Phase mit verd. Salzsäure und Wasser gewaschen, über Natriumsulfat getrocknet und unter vermindertem Druck eingeengt. Man erhält 172 mg Rohprodukt, das durch präparative Schichtchromatographie an Kieselgel PF ₂₄₅₊₃₆₆ mit dem Laufmittelgemisch Toluol/Aceton 4:1 gereinigt wird.

Es werden 115 mg 11β-[4-(Acetyloximinomethyl)phenyl]-17β-methoxy-17α-methoxymethyl-estra-4,9-dien-3-on erhalten. Das Produkt kristallisiert aus Essigester.
Schmelzpunkt: 115 - 120°C (Essigester)
α_{D} = + 218° (CHCl₃)
IR-Spektrum in KBr (cm⁻¹): 1754 (OAc);
1654 (C=C-C=C-C=0); 1602 (Phenyl)
UV-Spektrum in MeOH: λₘₐₓ = 271 nm ε = 28 157
λₘₐₓ = 297 nm ε = 26 369
¹H-NMR-Spektrum in CDCl₃ [δ, ppm]: 0,511 (s, 3H, H-18); 2,227 (s, 3H, OCOCH₃); 3,247 (s, 3H, 17β-OCH₃); 3,408 (s, 3H, 17α-CH₂OCH₃); 3,386, 3,431, 3,544, 3,580 (m, 2H, CH₂OCH₃); 4,399 (d, 1H, J=7,2 Hz, H-11α); 5,785 (s, 1H, H-4); 7,242, 7,266, 7,618, 7,647 (m, 4H, AA'BB'-System der Aromatenprotonen); 8,315 (s, 1H, CH=NOAc)
MS m/e: 446 C₂₈H₃₂NO₄ M⁺ - CH₂OCH₃ ⁺

### Beispiel 2

Zu 210 mg 11β-[4-(Hydroximinomethyl)phenyl]-17β-methoxy-17α-methoxymethyl-estra-4,9-dien-3-on werden in 5 ml Pyridin 0,3 ml Chlorameisensäureethylester unter Wasserkühlung getropft. Es entsteht ein weißer Niederschlag. Nach 30 Min. wird mit Wasser versetzt, es entsteht eine Lösung, danach fällt ein weißer Niederschlag aus, der abgesaugt und mit Wasser gewaschen wird. Ausbeute nach Trocknung 133 mg. Die wäßrige Phase wird mit Chloroform extrahiert, mit verd. Salzsäure und Wasser gewaschen, getrocknet und unter vermindertem Druck eingeengt. Ausbeute 66 mg. Beide Feststoffe werden vereinigt und durch präparative Schichtchromatographie an Kieselgel PF ₂₄₅₊₃₆₆ mit dem Laufmittelgemisch Toluol/Aceton 4:1 gereinigt.

Man erhält 150 mg 11β-[4-(Ethoxycarbonyloximinomethyl)phenyl]-17β-methoxy-17α-methoxymethyl-estra-4,9-dien-3-on, die aus Aceton/Hexan umkristallisiert werden.
Schmelzpunkt: 137 - 148°C
α_{D}= +204° (CHCl₃)
UV-Spektrum in MeOH: λₘₐₓ = 270 nm ε = 27 094
λₘₐₓ = 297 nm ε = 25 604
¹H-NMR-Spektrum in CDCl₃ [δ, ppm]: 0,507 (s, 3H, H-18); 1,383 (t, 3H, J=7,0 Hz, OCH₂CH₃); 3,246 (s, 3H, 17β-OCH₃); 3,410 (s, 3H, 17α-CH₂OCH₃); 3,39 - 3,56 (m, 2H, CH₂OCH₃); 4,35 (d, 1H, J=7,0 Hz, H-11α); 5,784 (s, 1H, H-4); 7,23, 7,26, 7,61, 7,64 (m, 4H, AA'BB'-System der Aromatenprotonen); 8,303 (s, 1H, CH=NR)
MS m/e: 431,24701 C₂₈H₃₃NO₃ M⁺ - C₂H₅OCOOH

### Beispiel 3

190 mg 11β-[4-(Hydroximinomethyl)phenyl]-17β-methoxy-17α-methoxymethyl-estra-4,9-dien-3-on werden in 10 ml Toluol suspendiert. Nacheinander werden 0,5 ml Phenylisocyanat und 1 ml Triethylamin zugegeben. Man rührt 3 Stunden bei Raumtemperatur, erhitzt 2 Stunden unter Rückfluß, saugt den weißen Niederschlag ab und engt das Lösemittel unter vermindertem Druck ein. Man erhält 310 mg eines hellbraunen Feststoffes, der durch präparative Schichtchromatographie an Kieselgel PF ₂₄₅₊₃₆₆ mit dem Laufmittelgemisch Toluol/Aceton 9:1 gereinigt wird.

Es werden 65 mg 17β-Methoxy-17α-methoxymethyl-11β-{4-[(phenylaminocarbonyl)oximinomethyl]phenyl}-estra-4,9-dien-3-on isoliert.
Schmelzpunkt: 241 - 246°C (Aceton)
α_{D}= +178°C (CHCl₃)
UV-Spektrum in MeOH: λₘₐₓ = 238 nm ε = 29 444
λₘₐₓ = 300 nm ε = 29 649
¹H-NMR-Spektrum in CDCl₃ [δ, ppm]: 0,474 (s, 3H, H-18); 3,245 (s, 3H, 17β-OCH₃); 3,405 (s, 3H, 17α-CH₂OCH₃); 3,406 - 3,545 (m, 2H, ABX-System, 17α-CH₂OCH₃); 4,413 (d, 1H, J=6,8 Hz, H-11α); 5,797 (s, 1H, H-4); 7,264 (m, 5H, Aromat); 7,272, 7,293, 7,548, 7,575 (m, 4H, AA'BB'-System der Aromatenprotonen); 8,0 (s, 1H, CH=N-).
MS m/e: 431,24249 C₂₈H₃₃NO₃ M⁺ - C₆H₅CNO + H₂O

### Beispiel 4

708 mg 11β-[4-(Hydroximinomethyl)phenyl]-17β-methoxy-17α-methoxymethyl-estra-4,9-dien-3-on werden in 15 ml Toluol gelöst. Nacheinander werden 1,5 ml Ethylisocyanat und 3 ml Triethylamin zugegeben. Man rührt 6 Stunden bei Raumtemperatur, läßt über Nacht stehen, fügt 20 ml wässr. Ammoniaklösung zu, trennt die Phasen, extrahiert mit Toluol nach, wäscht mit Wasser, wässr. Ammoniunchlorid-Lösung und Wasser, trocknet über Natriumsulfat und engt unter vermindertem Druck ein. Man erhält 800 mg eines hellgelben Feststoffes, der durch präparative Schichtchromatographie an Kieselgel 60 PF ₂₅₄₊₃₆₆ mit dem Laufmittelgemisch Toluol/Aceton (9:1) gereinigt wird.

Es werden 610 mg 11β-{4-[(Ethylaminocarbonyl)oximinomethyl]phenyl}-17β-methoxy-17α-methoxymethyl-estra-4,9-dien-3-on isoliert.
Schmelzpunkt: 142 - 147°C Zersetzung im Licht (Ether/Aceton/Hexan)
¹H-NMR-Spektrum in CDCl₃ [δ, ppm]: 0,522 (s, 3H, H-18); 1,241 (t, 3H, J=7,5 Hz, NHCH₂CH₃); 3,253 (s, 3H, 17β-OCH₃); 3,415 (s, 3H, 17α-CH₂OCH₃); 3,366 - 3,574 (m, 4H, ABX-System, 17α-CH₂OCH₃, NHCH₂CH₃); 4,410 (d, 1H, J=7,2 Hz, H-11α); 5,790 (s, 1H, H-4); 6,238 (m, 1H, NHCO); 7,258, 7,286, 7,561, 7,589 (m, 4H, AA'BB'-System der Aromatenprotonen); 8,294 (s, 1H, CH=N-)

### Beispiel 5

500 mg 11β-[4-(Hydroximinomethyl)phenyl]-17β-methoxy17α-methoxymethyl-estra-4,9-dien-3-on werden in 4 ml Propionsäureanhydrid/Pyridin 1:1 (v:v) 2,5 Stunden unter Schutzgas gerührt. Man gießt in Eiswasser ein, extrahiert die klebrige Masse mit Chloroform, wäscht die organische Phase mit verd. Salzsäure und Wasser, trocknet über Natriumsulfat und engt unter vermindertem Druck ein. Der hellgelbe Schaum wird durch Chromatographie gereinigt und aus Essigester umkristallisiert. Ausbeute 306 mg 11β-[4-(Propionyloximinomethyl)phenyl]-17β-methoxy-17α-methoxy methyl-estra-4,9-dien-3-on.
Schmelzpunkt: 110 - 114°C (Essigester)
¹H-NMR-Spektrum in CDCl₃ [δ, ppm]: 0,515 (s, 3H, H-18); 1,241 (t, 3H, J=7,6 Hz, OCOCH₂CH₃); 3,253 (s, 3H, 17β-OCH₃); 3,415 (s, 3H, 17α-CH₂OCH₃); 3,4 - 3,6 (m, 2H, ABX-System, 17α-CH₂OCH₃); 4,128 (d, 1H, J=7,2 Hz, H-11α); 5,790 (s, 1H, H-4); 7,244, 7,271, 7,627, 7,655 (m, 4H, AA'BB'-System der Aromatenprotonen); 8,322 (s, 1H, CH=N-)

### Beispiel 6

Zu 500 mg 11β-[4-(Hydroximinomethyl)phenyl]-17β-methoxy-17α-methoxymethyl-estra-4,9-dien-3-on wird eine filtrierte Mischung aus 2 ml Benzoylchlorid und 3 ml Pyridin gegeben. Nach 2 Stunden wird in 150 ml Eiswasser eingerührt. Das Steroid fällt als klebrige Masse aus. Man fügt 5 ml verd. Salzsäure zu, nimmt in Essigester auf, trennt die Phasen, wäscht die organische Phase mit wäßriger Bicarbonat-Lösung und Wasser, trocknet über Natriumsulfat, filtriert ab und verdampft unter vermindertem Druck. Das gelbe Öl (1,57 g) wird durch Chromatographie an 40 g Kieselgel 60 mit einem Toluol/Essigester-Gradienten von unpolaren Produkten befreit. Die Hauptfraktion (0,7 g) wird durch präparative Schichtchromatographie an Kieselgel 60 PF₂₅₄₊₃₆₆ mit dem Laufmittelgemisch Chloroform/Aceton gereinigt. Man erhält 410 mg als farblosen Schaum, der aus Methanol umkristallisiert wird.
Es werden 263 mg 11β-[4-(Benzoyloximinomethyl)phenyl]-17β-methoxy-17α-methoxymethyl-estra-4,9-dien-3-on als farblose Kristalle erhalten.
Schmelzpunkt: 115 - 122°C (Methanol)
α_{D} = + 216° (CHCl₃)
UV-Spektrum in MeOH: λₘₐₓ = 279 nm ε = 33 720
λₘₐₓ = 299 nm ε = 30 120
¹H-NMR-Spektrum in CDCl₃ [δ, ppm]: 0,527 (s, 3H, H-18); 3,253 (s, 3H, 17β-OCH₃); 3,415 (s, 3H, 17α-CH₂OCH₃); 3,403 - 3,589(m, 2H, ABX-System, 17α-CH₂OCH₃); 4,416 (d, 1H, J=7,2 Hz, H-11α); 5,792 (s, 1H, H-4); 7,299 - 7,615 (m, 5H, Aromatenprotonen, COPhenyl); 7,701, 7,729, 8,111, 8,140 (m, 4H, AA'BB'-System der Aromatenprotonen); 8,520(s, 1H, CHPhenyl)

### Beispiel 7

### Messung der Rezeptor-Bindungsaffinität:

Die Rezeptorbindungsaffinität wurde bestimmt durch competitive Bindung eines spezifisch bindenden ³H-markierten Hormons (Tracer) und der zu testenden Verbindung an Rezeptoren im Cytosol aus tierischen Target-Organen. Dabei wurden Rezeptorsättigung und Reaktionsgleichgewicht angestrebt. Folgende Inkubationsbedingungen wurden gewählt:

Glucocorticoid-Rezeptor: Thymus-Cytosol der adrenalectomierten Ratte, Thymi aufbewahrt bei -30°C; Puffer: TED. Tracer: ³H-Dexamethason, 20 nM; Referenzsubstanz: Dexamethason.

Nach einer Inkubationsdauer von 18 Stunden bei 0 - 4°C erfolgte die Trennung von gebundenem und freiem Steroid durch Einmischen von Aktivkohle/Dextran (1%/0,1%), Abzentrifugieren und Messung der gebundenen ³H-Aktivität im Überstand.

Aus Messungen in Konzentrationreihen wurden die IC₅₀ für die zu testende Verbindung und für die Referenzsubstanz ermittelt und als Quotient beider Werte (x 100%) die relative molare Bindungsaffinität.

### Beispiel 8

Hemmung der frühen Gravidität bei der Ratte: Rattenweibchen werden im Prooestrus angepaart. Bei Nachweis von Spermien im Vaginalabstrich am nächsten Tag wird dieser Tag als Tag 1 (=d1) der Gravidität gerechnet. Die Behandlung mit Testsubstanz oder Vehikel erfolgt von d5 - d7, die Autopsie erfolgt d9. Die Substanzen werden in 0,2 ml Vehikel (Benzylbenzoat/Rizinusöl 1 + 4) subcutan injiziert. Die Rate vollständig gehemmter Graviditäten in den verschiedenen Gruppen ergibt sich aus Tabelle 1. Es wurde für J 917 und J 900 eine dem RU 486 überlegene Nidationshemmung festgestellt.

## Patentansprüche

1. 11β-Benzaldoxim-estra-4,9-dien-Derivate der allgemeinen Formel I, 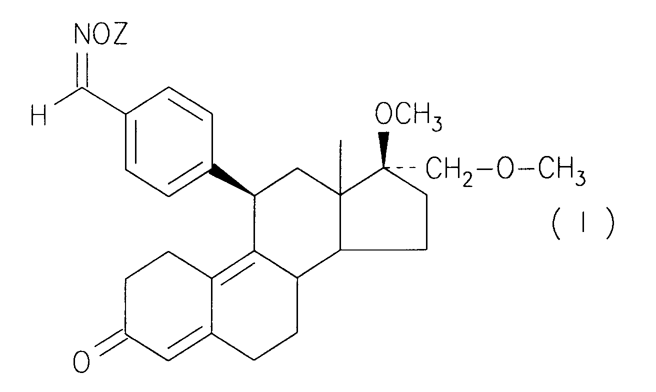 worin
Z für -CO-CH₃, -CO-O-C₂H₅, -CO-NH-Phenyl, -CO-NH-C₂H₅, -CO-C₂H₅ oder -CO-Phenyl steht,
sowie deren pharmazeutisch annehmbaren Salze.

2. Verbindungen nach Anspruch 1, nämlich
11β-[4-(Acetoximinomethyl)phenyl]-17β-methoxy-17α-methoxymethyl-estra-4,9-dien-3-on,
11β-{4-[(Ethoxycarbonyl)oximinomethyl]phenyl}-17β-methoxy-17α-methoxymethyl-estra-4,9-dien-3-on,
11β-{4-[(Ethylaminocarbonyl)oximinomethyl]phenyl}-17β-methoxy-17α-methoxymethyl-estra-4,9-dien-3-on,
17β-Methoxy-17α-methoxymethyl-11β-{4-[(phenylaminocarbonyl)oximinomethyl]phenyl}-estra-4,9-dien-3-on,
11β-[4-(Propionyloximinomethyl)phenyl]-17β-methoxy-17α-methoxymehyl-estra-4,9-dien-3-on, und
11β-[4-(Benzoyloximinomethyl)phenyl]-17β-methoxy-17α-methoxymethyl-estra-4,9-dien-3-on.

3. Verfahren zur Herstellung der Verbindungen nach Anspruch 1 und deren pharmazeutisch annehmbaren Salze, dadurch gekennzeichnet, daß man eine Verbindung der allgemeinen Formel II, 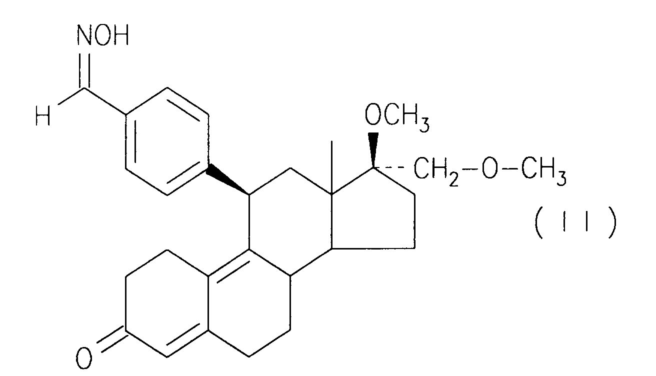 mit der Gruppe Z verestert und gegebenenfalls die erhaltene Verbindung in ein pharmazeutisch annehmbares Salz überführt.

4. Pharmazeutische Zusammensetzungen, gekennzeichnet durch den Gehalt an mindestens einer Verbindung nach einem der Ansprüche 1 bis 2.

## Claims

1. 11β-benzaldoxime-estra-4,9-diene derivatives of the general formula I 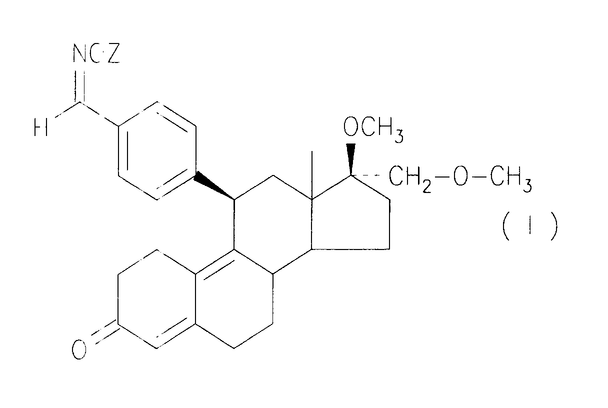 where
Z is -CO-CH₃, -CO-O-C₂H₅, -CO-NH-phenyl, -CO-NH-C₂H₅, -CO-C₂H₅, or CO-phenyl,
as well as their pharmaceutically acceptable salts.

2. Compounds according to Claim 1, namely
11β-[4-(acetoximinomethyl)phenyl]-17β-methoxy-17α-methoxymethyl-estra-4,9-diene-3-on,
11β-{4-[(ethoxycarbonyl)oximinomethyl]phenyl}-17β-methoxy-17α-methoxymethyl-estra-4,9-diene-3-on,
11β-{4-[(ethylaminocarbonyl)oximinomethyl]phenyl}-17β-methoxy-17α-methoxymethyl-estra-4,9-diene-3-on,
17β-methoxy-17α-methoxymethyl-11β-{4-[(phenylaminocarbonyl)oximinomethyl]phenyl}-estra-4,9-diene-3-on,
11β-[4-(propionyloximinomethyl)phenyl]-17β-methoxy-17α -methoxymethyl-estra-4,9-diene-3-on, and
11β-[4-(benzoyloximinomethyl)phenyl]-17β-methoxy-17α-methoxymethyl-estra-4,9-diene-3-on.

3. Methods for producing the compounds according to claim 1 and their pharmaceutically acceptable salts, characterized in that a compound of the general formula II 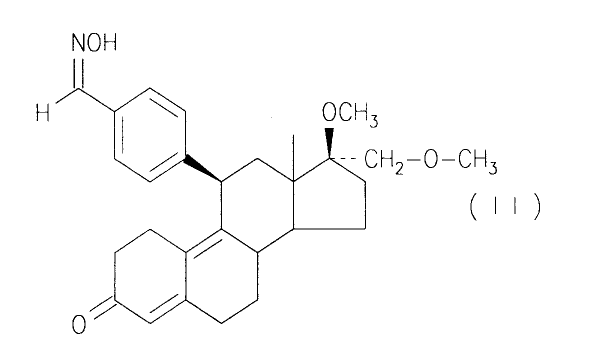 is esterified with the group Z and the resulting compound is optionally converted into a pharmaceutically acceptable salt.

4. Pharmaceutical compositions, characterized in that they contain at least one compound according to claims 1 to 2.

## Revendications

1. Dérivé 11β-benzaldoxim-estra-4,9-diène de formule générale I 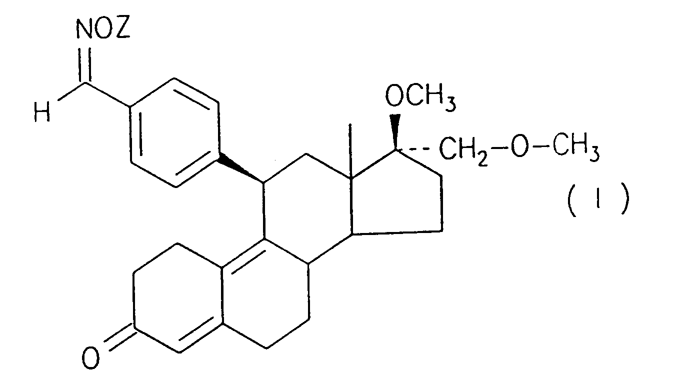 dans laquelle
Z représente -CO-CH₃, -CO-O-C₂H₅, -CO-NH-phényle, -CO-NH-C₂H₅, -CO-C₂H₅ ou -CO-phényle,
ainsi que ses sels pharmaceutiquement acceptables.

2. Composé selon la revendication 1, en particulier 11β-[4-(acétoximinométhyl)phényl]-17β-méthoxy-17α-méthoxyméthyl-estra-4,9-diène-3-one,
11β-{4-(éthoxycarbonyl)oximinométhyl]phényl}-17β-méthoxy-17α-méthoxyméthyl-estra-4,9-diène-3-one,
11β-{4-(éthylaminocarbonyl)oximinométhyl]phényl}-17β-méthoxy-17α-méthoxyméthyl-estra-4,9-diène-3-one,
17β-méthoxy-17α-méthoxyméthyl-11β-{4-(phénylaminocarbonyl)oximinométhyl]phényl}-estra-4,9-diène-3-one,
11β-[4-(propionyloximinométhyl)phényl]-17β-méthoxy-17α-méthoxyméthyl-estra-4,9-diène-3-one,
11β-[4-(benzoyloximinométhyl)phényl]-17β-méthoxy-17α-méthoxyméthyl-estra-4,9-diène-3-one.

3. Procédé de préparation des composés selon la revendication 1 et leurs sels pharmaceutiquement acceptables, caractérisé en ce que l'on estérifie un composé de formule générale II, 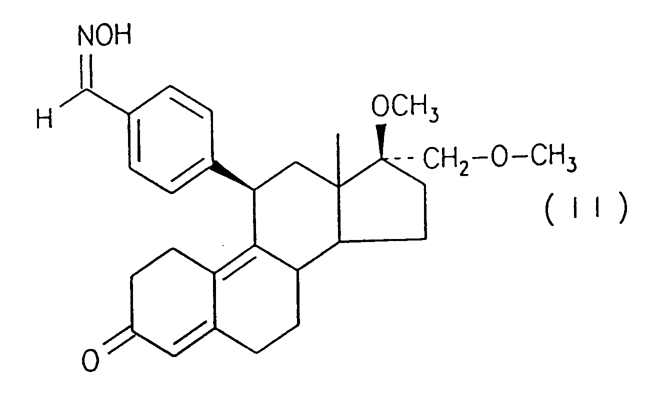 avec le groupe Z, et éventuellement on transforme le composé obtenu en un sel pharmaceutiquement acceptable.

4. Composition pharmaceutique, caractérisée en ce qu'elle contient au moins un composé selon l'une quelconque des revendications 1 à 2.
